(19) Europäisches Patentamt European Patent Office Office européen des brevets

(11) **EP 3 892 226 A1**

(12) **EUROPEAN PATENT APPLICATION**
published in accordance with Art. 153(4) EPC

(43) Date of publication:
**13.10.2021 Bulletin 2021/41**

(51) Int Cl.:
***A61B 34/30*** (2016.01)     ***A61B 34/00*** (2016.01)

(21) Application number: 19893425.9

(22) Date of filing: 18.11.2019

(86) International application number:
**PCT/CN2019/119247**

(87) International publication number:
**WO 2020/114233 (11.06.2020 Gazette 2020/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 04.12.2018 CN 201811472969

(71) Applicant: **Shanghai Microport Medbot (Group) Co., Ltd.**
**Shanghai 201203 (CN)**

(72) Inventors:
• **LI, Zihan**
**Shanghai 201203 (CN)**

• **WANG, Jiayin**
**Shanghai 201203 (CN)**
• **HE, Chao**
**Shanghai 201203 (CN)**
• **NI, Feijian**
**Shanghai 201203 (CN)**
• **ZHANG, Peng**
**Shanghai 201203 (CN)**
• **XIA, Yuhui**
**Shanghai 201203 (CN)**

(74) Representative: **Pediani, Steven Peter**
**Marks & Clerk LLP**
**1 New York Street**
**Manchester M1 4HD (GB)**

(54) **METHOD AND SYSTEM FOR PREVENTING COLLISION BETWEEN MECHANICAL ARMS, AND MEDICAL ROBOT**

(57) A method and system for preventing a collision between mechanical arms (21), and a medical robot, belonging to the field of medical robot technology. The method includes: arranging (S10) discrete points ($m$, $n$) at a mechanical arm (21); acquiring (S40) an interaction force ($F_{m,n}$) corresponding to each discrete point ($m$, $n$) according to a calculated relative distance (L) between the discrete points ($m$, $n$) respectively on different mechanical arms (21), to obtain (S50) a resultant force of the interaction forces ($F_{m,n}$) each of which corresponds to each discrete point ($m$, $n$), and then obtaining a Cartesian force ($F_d$) corresponding to each mechanical arm (21), and making (S60) an operator perceive the Cartesian force ($F_d$) in real time, thereby effectively reducing the risk of interference and collision between the mechanical arms (21).

FIG. 12

## Description

### CROSS REFERENCE TO RELATED APPLICATION

This application claims priority to Chinese Patent Application with No. 201811472969.3, entitled "Method and System for Preventing Collision Between Mechanical Arms, and Medical Robot", and filed on December 4, 2018, the content of which is expressly incorporated herein by reference in its entirety.

### TECHNICAL FIELD

The present disclosure relates to field of medical robot technology, and particularly to a method and a system for preventing a collision between mechanical arms, and a medical robot.

### BACKGROUND

Currently, a medical robot generally needs to control a plurality of mechanical arms to cooperate with each other during a specific operation. However, during the movements of the mechanical arms, an interference, a collision and other defects occur easily between the mechanical arms.

For example, in the process of performing related operations such as detection and surgery, a teleoperation medical robot generally controls more than two mechanical arms, such as tool arms, to work collaboratively, so that a medical instrument carried by each arm can be moved to an expected position, and then operations such as detection or surgery are performed for the focus. However, during the movements of the mechanical arms, due a small distance between the mechanical arms, risks of interference and collision can easily occur between the mechanical arms, and then the operations such as the detection and surgery can be affected, which may even cause medical accidents in serious cases.

### SUMMARY

Various exemplary embodiments in the present disclosure provide a method and a system for preventing a collision between mechanical arms, and a medical robot.

In an optional embodiment, the present disclosure provides a method for preventing a collision between mechanical arms, applied to a medical robot, the medical robot including at least two mechanical arms, a mechanical arm including a mechanical arm body, the mechanical arm body including a plurality of joints, the method including:

arranging a plurality of discrete points on each mechanical arm;
acquiring first coordinate information of each discrete point in a global coordinate system;
obtaining a first relative distance between two discrete points located on different mechanical arms according to the first coordinate information;
acquiring a first interaction force corresponding to each discrete point and each of discrete points on the other mechanical arm according to the first relative distance and a corresponding relationship between the first relative distance and the first interaction force; and
obtaining a Cartesian force of each mechanical arm according to a resultant force of first interaction forces each of which corresponds to each discrete point and each of discrete points on the other mechanical arm, and making the Cartesian force perceived by an operator.

In the above embodiment of the method for preventing the collision between mechanical arms, discrete points are arranged on the mechanical arm, and the forces at each discrete point are acquired according to the calculated distance between the discrete points on different mechanical arms, to obtain the resultant force of the interaction forces corresponding to each discrete point, and then the Cartesian force of each mechanical arm is obtained, and the operator can perceive the Cartesian force in real time, thereby effectively avoiding defects such as interference and collisions between the mechanical arms.

In an optional embodiment, at least a part of the mechanical arms further includes a medical instrument mounted on a distal end of the mechanical arm body, the arranging the plurality of discrete points on each mechanical arm includes arranging the discrete points on a medical instrument and/or the mechanical arm body.

In an optional embodiment, the arranging the plurality of discrete points on each mechanical arm includes: acquiring first relative position information of a discrete point with respect to a corresponding joint.

In an optional embodiment, the mechanical arm further includes a position sensor configured to acquire joint position information, the acquiring the first coordinate information of each discrete point in the global coordinate system includes:

obtaining the joint position information of the joint by the position sensor, and obtaining second coordinate information of each joint in the global coordinate system according to the joint position information and a kinematic model; and obtaining the first coordinate information of the discrete point in the global coordinate system according to the second coordinate information and the first relative position information.

In an optional embodiment, the acquiring the first interaction force corresponding to each discrete point and each of discrete points on the other mechanical arm according to the first relative distance and the corresponding relationship between the first relative distance and the first interaction force includes:

setting a plurality of distance intervals in a value range of the first relative distance, the corresponding relationship corresponding to at least one of the distance intervals being different from corresponding relationships corresponding to other distance intervals; and obtaining the first interaction force corresponding to each discrete point and each of discrete points on the other mechanical arm according to the first relative distance between the discrete points and the corresponding relationship corresponding to a distance interval in which the first relative distance is.

In an optional embodiment, the plurality of distance intervals includes a first distance interval (0, $L_{min} + R_m + R_n$] , a second distance interval $(L_{min} + R_m + R_n, L_{max} + R_m + R_n)$, and a third distance interval $[L_{max} + R_m + R_n, +\infty)$; and

the first interaction force F is a preset maximum interaction force $F_{max}$ in the first distance interval (0, $L_{min} + R_m + R_n$] ; a relationship between the first interaction force and the first relative distance is a function in the second distance interval $(L_{min} + R_m + R_n, L_{max} + R_m + R_n)$, the function is a continuous function in the second distance interval, a first derivative of the function is less than zero, and a second derivative of the function is greater than zero; the first interaction force F is equal to zero in the third distance interval $[L_{max} + R_m + R_n, +\infty)$; discrete points $m$ and $n$ are two discrete points on different mechanical arms, $R_m$ is a cross-sectional maximum radius of a mechanical arm at the discrete point $m$, with the discrete point $m$ located on the mechanical arm; $R_n$ is a cross-sectional maximum radius of a mechanical arm at the discrete point $n$, with the discrete point $n$ located on the mechanical arm; $L_{max}$ is a preset collision warning distance of the first relative distance, and $L_{min}$ is a preset minimum safe distance of the first relative distance.

In an optional embodiment the function is:

$$ f(L) \;=\; k(\frac{1}{L} - \frac{1}{L_{\max}}) \,(\frac{1}{L^2})L' , $$

$k$ is a distance-force gain coefficient, $L$ is the first relative distance, and $L'$ is a derivative of $L$ with respect to time.
In an optional embodiment, the arranging the plurality of discrete points on each mechanical arm further includes:

acquiring a discrete point gain coefficient of each discrete point;
the obtaining the Cartesian force of each mechanical arm according to the resultant force of the first interaction forces each of which corresponds to each discrete point and each of discrete points on the other mechanical arm includes:
obtaining a resultant force of all first interaction forces corresponding to each discrete point;
obtaining a torque corresponding to each discrete point with respect to a corresponding joint according to the resultant force of all the first interaction forces corresponding to each discrete point and the first relative position information of each discrete point with respect to a corresponding joint, and obtaining a resultant torque corresponding to each joint according to a discrete point gain coefficient of each discrete point; and
obtaining the Cartesian force of the mechanical arm according to the resultant torque corresponding to each joint and a force Jacobian matrix of the mechanical arm.

In an optional embodiment, the making the Cartesian force perceived by the operator includes:
displaying the Cartesian force by a display device.
In an optional embodiment, the mechanical arm is connected to a master manipulator configured to control a movement of the mechanical arm, the making the Cartesian force perceived by the operator includes:

obtaining a Cartesian force of the master manipulator configured to control the movement of the mechanical arm according to the Cartesian force of the mechanical arm;

obtaining a torque corresponding to each joint of the master manipulator according to a force Jacobian matrix and the Cartesian force of the master manipulator; and

causing a motor configured to control a movement of a joint of the master manipulator to correspondingly output a resistance torque having a same magnitude as but an opposite direction to the torque corresponding to each joint of the master manipulator.

In an optional embodiment, the present disclosure also provides a system for preventing a collision between mechanical arms, applied to a medical robot, the medical robot includes at least two mechanical arms, a mechanical arm includes a mechanical arm body, the mechanical arm body includes a plurality of joints, the system includes:

a discrete point arranging unit configured to arrange a discrete point on the mechanical arm;
a memory configured to store instructions;
a processor in communication with the discrete point arranging unit and the memory, respectively;
the instructions stored in the memory, when executed by the processor, perform the steps of:
acquiring first coordinate information of each discrete point in a global coordinate system;
obtaining a first relative distance between two discrete points on different mechanical arms according to the first coordinate information;
acquiring a first interaction force corresponding to each discrete point and each of discrete points on the other mechanical arm according to the first relative distance and a corresponding relationship between the first relative distance and the first interaction force; and
obtaining a Cartesian force of each mechanical arm according to a resultant force of first interaction forces each of which corresponds to each discrete point and each of discrete points on the other mechanical arm, and making the Cartesian force perceived by an operator.

In an optional embodiment, at least a part of the mechanical arms further includes a medical instrument mounted on a distal end of the mechanical arm body, and the discrete point is arranged on the medical instrument and/or the mechanical arm body.

In an optional embodiment, the mechanical arm further includes a position sensor configured to acquire joint position information, the position sensor being in communication with the processor;

the discrete point arranging unit is further configured to acquire first relative position information of the discrete point with respect to a corresponding joint;
the instructions stored on the memory, when executed by the processor, further perform the steps of:
acquiring, by the position sensor, joint position information of the joint, and acquiring second coordinate information of each joint in the global coordinate system according to the joint position information and a kinematic model; and
acquiring the first coordinate information of each discrete point in the global coordinate system according to the second coordinate information of each joint in the global coordinate system and the first relative position information.

In an optional embodiment, the corresponding relationship between the first relative distance and the first interaction force is pre-stored in the memory;

or,
the system further includes an input device configured to acquire the corresponding relationship between the first relative distance and the first interaction force during a surgery, to directly be executed by the processor or stored in the memory.

In an optional embodiment, a value range of the first relative distance includes a plurality of distance intervals, and the corresponding relationship corresponding to at least one of the distance intervals is different from corresponding relationships corresponding to other distance intervals;
the instructions stored on the memory, when executed by the processor, further perform the steps of:
obtaining a first interaction force corresponding to each discrete point and each of discrete points on the other mechanical arm according to the first relative distance of each discrete point and a corresponding relationship corresponding to a distance interval in which the first relative distance is.

In an optional embodiment, the preset plurality of distance intervals include a first distance interval $(0, L_{min} + R_m + R_n]$, a second distance interval $(L_{min} + R_m + R_n, L_{max} + R_m + R_n)$, and a third distance interval $[L_{max} + R_m + R_n, +\infty)$; and

the first interaction force F is a preset maximum interaction force $F_{max}$ in the first distance interval (0, $L_{min} + R_m + R_n$] ; a relationship between the first interaction force and the first relative distance is a function in the second distance interval ($L_{min} + R_m + R_n$, $L_{max} + R_m + R_n$), the function is a continuous function in the second distance interval, and a first derivative of the function is less than zero, and a second derivative of the function is greater than zero; the first interaction force F is zero in the third distance interval $[L_{max} + R_m + R_n$, +∞);

discrete points m and n are two discrete points on different mechanical arms, $R_m$ is a cross-sectional maximum radius of a mechanical arm at the discrete point m, with the discrete point m located on the mechanical arm; $R_n$ is a cross-sectional maximum radius of a mechanical arm at the discrete point n, with the discrete point n located on the mechanical arm; $L_{max}$ is a preset collision warning distance of the first relative distance, and $L_{min}$ is a preset minimum safe distance of the first relative distance.

In an optional embodiment, the function is:

$$f(L) = k(\frac{1}{L} - \frac{1}{L_{max}}) (\frac{1}{L^2})L',$$

k is a distance-force gain coefficient, L is the first relative distance, and L' is a derivative of L with respect to time.

In an optional embodiment, the discrete point arranging unit is further configured to obtain a discrete point gain coefficient of each discrete point;

the instructions stored in the memory, when executed by the processor, further perform steps of:

obtaining a resultant force of first interaction forces each of which corresponds to each discrete point and each of discrete points on the other mechanical arm;

obtaining a torque corresponding to each discrete point with respect to a corresponding joint according to the resultant force of the first interaction forces corresponding to each discrete point and the first relative position information of each discrete point with respect to a corresponding joint, and obtaining a resultant torque corresponding to each joint according to the torque corresponding to the discrete point with respect to the corresponding joint and the discrete point gain coefficient of each discrete point; and

obtaining a Cartesian force of the mechanical arm according to the resultant torque corresponding to each joint and a force Jacobian matrix of the mechanical arm.

In an optional embodiment, the system further includes an alarm connected to the processor, the instructions stored on the memory, when executed by the processor, further perform steps of:

when a first relative distance of any discrete point on the mechanical arm is less than or equal to a preset warning distance, and/or when the Cartesian force of the mechanical arm is greater than a preset force threshold, triggering the alarm to send out a warning message.

In an optional embodiment, the system further includes:

a display apparatus, the processor further configured to display the Cartesian force through the display device.

In an optional embodiment, a medical robot includes:

at least two mechanical arms, a mechanical arm includes a mechanical arm body, the mechanical arm body includes a plurality of joints; and

the system for preventing the collision between mechanical arms according to any one of the above embodiments.

In an optional embodiment, the medical robot further includes a physician side, a patient side, and a control end;

the physician side, the patient side, and the system for preventing the collision between mechanical arms are respectively in communication with the control end; the mechanical arm is located at the patient side; the physician side includes a master manipulator, the master manipulator includes a plurality of joints and a motor driving the joint to move, the master manipulator is configured to control a movement of the mechanical arm; the control end is configured to set a Cartesian force of the master manipulator according to a Cartesian force of the mechanical arm obtained from the system for preventing the collision between the mechanical arms; and

the control end is further configured to obtain a torque corresponding to each joint of the master manipulator according to the Cartesian force and a force Jacobian matrix of the master manipulator, and control the motor to output a resistance torque having a same magnitude as but an opposite direction to the torque, to make the Cartesian force of the mechanical arm perceived by an operator.

## BRIEF DESCRIPTION OF THE DRAWINGS

In order to more clearly describe the technical solution in the embodiments or the prior art, the accompanying drawings needing to be used in the description of the embodiment or the prior art will be briefly introduced below. Apparently, the drawings in the following description are merely some embodiments of the present disclosure, and those of ordinary skill in the art can obtain drawings of other embodiments based on these drawings without creative work.

FIG. 1 is a schematic structure diagram of a control end in a teleoperation medical robot according to an optional embodiment.

FIG. 2 is a schematic structure diagram of a patient side in a teleoperation medical robot according to an optional embodiment.

FIG. 3 is a schematic diagram of a module structure of a teleoperation medical robot according to an optional embodiment.

FIG. 4 is a schematic diagram of a module structure of a system for preventing a collision between mechanical arms according to an optional embodiment.

FIG. 5 is a schematic diagram of arranging discrete points by a discrete point arranging unit according to an optional embodiment.

FIG. 6 is a schematic curve diagram illustrating a relationship between a first relative distance and a first interaction force according to an optional embodiment.

FIG. 7 is a schematic diagram of arranging discrete points for a surgical instrument in a teleoperation medical robot according to an optional embodiment.

FIG. 8 is a schematic diagram illustrating a section A-A at a discrete point 404x along a line A shown in FIG. 7.

FIG. 9 is a schematic diagram illustrating a pose of a mechanical arm of a teleoperation medical robot according to an optional embodiment.

FIG. 10 is a schematic diagram illustrating a force on each discrete point in FIG. 9.

FIG. 11 is a schematic diagram illustrating a force on a master manipulator in a teleoperation medical robot according to an optional embodiment.

FIG. 12 is a flow chart showing a method for preventing a collision between mechanical arms according to an optional embodiment.

## DETAILED DESCRIPTION

In order to make the objectives, technical solution, and advantages of the present disclosure clearer, the disclosure will be further described in detail with reference to the accompanying drawings and embodiments. It should be appreciated that the specific embodiments described here are only used for explaining the present disclosure, rather than limiting the present disclosure.

In the present disclosure, for each component, one end adjacent to the patient is a distal end or rear end; and the other end is a proximal end or front end.

The technical contents of the present disclosure are described in detail below by taking a teleoperation medical robot as an example in combination with the actual situations.

Teleoperation medical robots generally include components such as a control end, a physician side, and a patient side. Both the physician side and the patient side are in communication with the control end, that is, the physician side controls a relevant medical equipment on the patient side through the control end to perform a corresponding detection or surgery and the like. Generally, more than three mechanical arms are installed on the patient side, and the mechanical arm can include a mechanical arm body (such as a tool arm) and a position sensor. The mechanical arm body can include a plurality of joints and connecting rods connected through the joints; and the position sensor is configured to obtain position information of each joint (for example, a rotation angle of a rotational joint, a movement displacement of a prismatic joint, etc.). At least part of the robotic arms can also include a medical instrument, such as a surgical instrument, or a detection instrument, mounted on the rear end of the above-mentioned robotic arm body; and the physician side can be provided with a master manipulator, that is, an operator can control the detection instrument through the master manipulator in a master-slave manner to acquire a surgical environment, and can control the mechanical arm body and/or surgical instrument through the main manipulator in the master-slave manner to perform collaborative surgical operations, etc.

It should be noted that, unless otherwise stated in the context, forces and torques of the relevant medical instruments (e.g., medical instruments such as the mechanical arms, surgical instruments mounted on the rear end of the mechanical arm body, detection instruments, etc.) acquired and related to the patient side are virtual physical quantities, i.e., the forces and torques are not actually physically applied between the relevant mechanical arms of the patient side, but are merely parameter values acquired through specific algorithms and/or procedures to describe the urgency of the collision between the mechanical arms. In comparison, the actual physical quantities of the force and torque acquired in relation to the patient side which can represent the urgency of the collision between the mechanical arms may also be virtual physical quantities, i.e., the force and torque here can be perceived by touch from the operator through a specific device (e.g., a master manipulator), or can be perceived by vision or auditory from the operator through a specific device (e.g., a display device or a speaker), which is not limited herein.

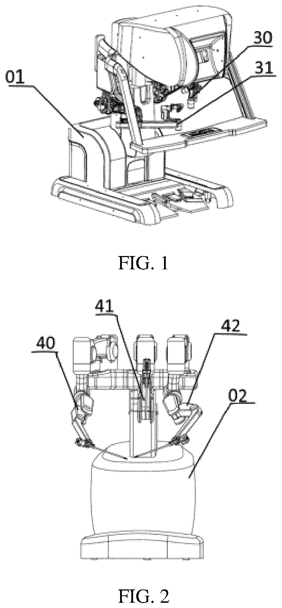

FIG. 1 is a schematic structure diagram of a physician side in a teleoperation medical robot according to an optional embodiment; and FIG. 2 is a schematic structure diagram of a patient side in a teleoperation medical robot according to an optional embodiment. As shown in FIGS. 1-2, the teleoperation medical robot can include a console 01 at the physician side and a platform 02 at the patient side; and a first master manipulator 30 and a second manipulator 31 are provided on the console 01. A first mechanical arm body 40, a second mechanical arm body 41, and a third mechanical arm body 42 are provided on the platform 02; and a medical instrument such as a surgical instrument or a detection instrument can be mounted on each mechanical arm body. At the same time, each mechanical arm body can also be configured to drive the medical instrument such as the surgical instrument or the detection instrument mounted on each mechanical arm body to move around a fixed point. According to a mapping relationship between the movement of the master manipulator and the movement of the mechanical arm, the movement of the mechanical arm can be controlled by operating the master manipulator, to control the detection instrument to detect and observe the surgical environment of a target position and control the surgical instrument to perform corresponding processing on the target position. The particular configuration of the mechanical arm body and the particular type of medical instrument are not specifically limited in the exemplary embodiments of the present disclosure.

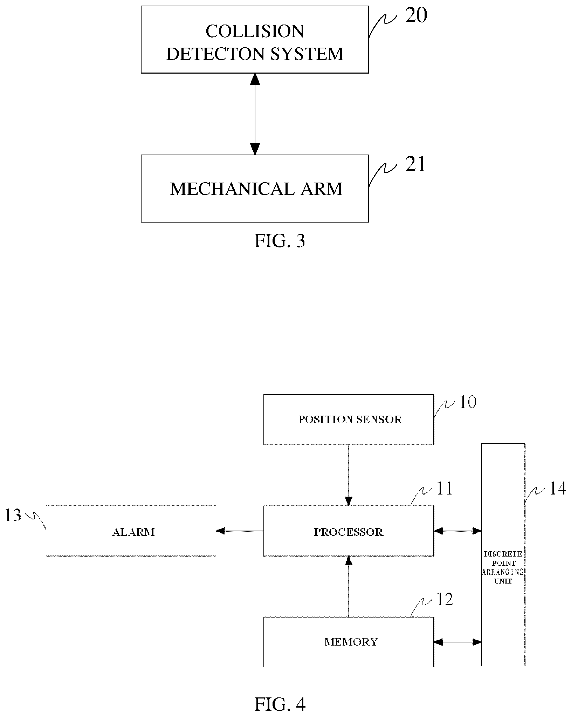

FIG. 3 is a schematic diagram illustrating a module structure of a teleoperation medical robot according to an optional embodiment. As shown in FIG. 3, the teleoperation medical robot can further include a system for preventing a collision between mechanical arms which can be configured to perform an anti-collision operation of the mechanical arm 21 of the teleoperation medical robot in the above embodiments. When the teleoperation medical robot is operated, it is possible to effectively prevent mutual collision between the mechanical arms, and enable the operator to perceive the movement condition of the mechanical arms in real time by feeding back the detected collision information of the mechanical arms to the operator in a force manner, which will be described in more detail below.

FIG. 4 is a schematic diagram illustrating a module structure of a system for preventing a collision between mechanical arms according to an optional embodiment. As shown in FIGS. 3-4, a position sensor 10 capable of acquiring position information of each joint is provided on the mechanical arm 21. The system 20 for preventing a collision can include components such as a processor 11, a memory 12, and a discrete point arranging unit 14. The memory 12 is configured to store instructions or code programs that, when executed by the processor, can cause the system to perform specific processes. The processor 11 can be in communication with the position sensor 10, the memory 12 and the discrete point arranging unit 14 of each joint, respectively. Here, the discrete point arranging unit 14 can be configured to arrange a discrete point on a mechanical arm body, such as a tool arm, and/or a medical instrument. The discrete point arranging unit 14 can also acquire discrete point information such as a first relative position of each discrete point with respect to a corresponding joint and a discrete point gain coefficient of each discrete point.

In some embodiments, the memory 12 can store information data such as the information of the relationship between the first relative distance and the first interaction force, information of the size of each mechanical arm, and information of the discrete point. The above information can be stored in the memory 12 in advance, for example, before the surgery, at the time of system initialization or leaving the factory, or the information can be inputted temporarily through an input device during the surgery, and is directly acquired by the processor 11 to perform, or is stored in the memory 12. The information of the size of the mechanical arm can include information of a size of the mechanical arm body, information of a size of the medical instrument (such as a surgical instrument and/or a detection instrument), and the like. Further, when the discrete points are arranged on the mechanical arm, the number and distribution of the discrete points can be determined according to the force collision detection sensitivity, the surgical operation requirements, and the size of each component of the mechanical arm.

In another optional embodiment, the discrete point arranging unit 14 can arrange discrete points on the mechanical arm body (e.g., tool arm) and/or the medical instrument according to an instruction issued by the processor 11. At the same time, the processor 11 can also output the discrete point information such as a first relative position of each discrete point with respect to a corresponding joint, a discrete point gain coefficient of each discrete point, and the like to the memory 12 for subsequent retrieval by the processor 11 or other devices. In an alternative solution, the memory 12 can also be in communication with the discrete point arranging unit 14, and can also be configured to store discrete point arranging rule information. The discrete point arranging unit 14 can arrange the discrete points on the mechanical arm body (e.g., the tool arm) and/or the medical instrument according to the retrieved discrete point arranging rule information pre-stored in the memory 12. At the same time, the processor 11 can also store the discrete point information such as a first relative position of each discrete point with respect to a corresponding joint, a discrete point gain coefficient of each discrete point, and the like into the memory 12 for subsequent retrieval by the processor 11 or other devices.

In an optional embodiment, the processor 11 can be further configured to obtain first coordinate information of each discrete point in a global coordinate system according to the second coordinate information of the joints on each mechanical arm in the global coordinate system and the first relative position of the discrete point with respect to the corresponding joint, and obtain the first relative distance between the discrete points according to the first coordinate information of each discrete point, and obtain the first interaction force and a resultant force corresponding to each discrete point based on the corresponding relationship between the first interaction force and the first relative distance between the discrete points. The resultant force can be a resultant force of all first interaction forces corresponding to each discrete point. For any one mechanical arm, the processor 11 can obtain a Cartesian force (i.e., Spatial Force) of the mechanical arm based on the resultant forces corresponding to all discrete points on the mechanical arm and make the Cartesian force perceived by the operator.

In another optional embodiment of the present disclosure, the discrete point arranging unit 14 in the embodiment of the present disclosure can also be implemented by software, hardware, or a combination of software and hardware, and integrated with the processor 11; or functions of the processor 11 and the discrete point arranging unit 14 can be simultaneously implemented by one processing device.

It should be noted that the discrete points in the embodiment of the present disclosure can be generated by the system according to a certain rule (for example, the rule may include interval setting information, and then the discrete points can be arranged according to the interval setting information, and the size of the interval can be set according to precision and operation requirements), or can be generated by inputting the rule information by the operator and directly reading the inputted rule information by the processor 11. Each discrete point herein can be configured to represent one volume block on a connecting rod of the mechanical arm, and the discrete point is located at a geometric center of a corresponding volume block. Thus, the greater the number of the discrete points, the greater the number of the volume blocks the discrete points represent, and thus the smaller the volume of each volume block. The relative size of the volume block can be represented by the discrete point gain coefficient $g_i$. Where, $\Sigma\, g_i = 1$, $i = 1, 2, ... , j$, $j$ is the number of all discrete points on a connecting rod of a mechanical arm, and $j, j$ are positive integer, and $i < j$.

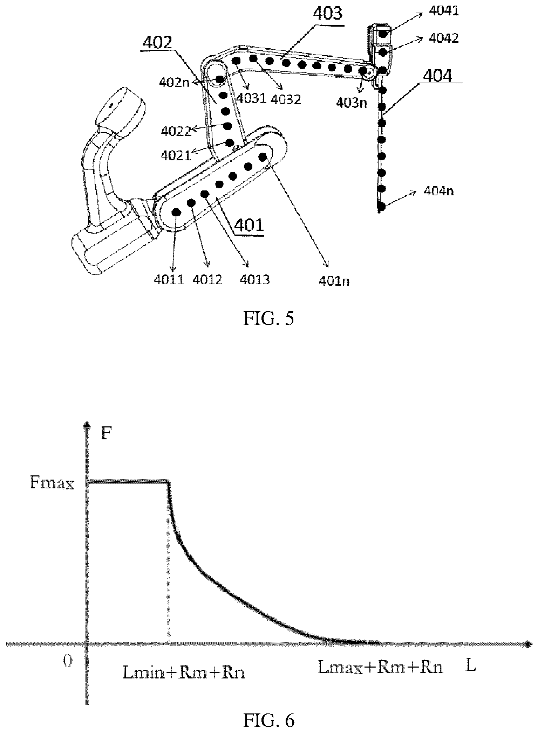

FIG. 5 is a schematic diagram of arranging discrete points by a discrete point arranging unit according to an optional embodiment. As shown in FIGS. 3-5, the mechanical arm body of the first mechanical arm 40 can include a first large arm 401, a first middle arm 402, and a first small arm 403 which are sequentially connected by joints. A first surgical instrument 404 is mounted on the distal end of the mechanical arm body of the first mechanical arm 40 (i.e., the first small arm 403). A discrete points (i.e., a discrete point 4011, a discrete point 4012, ..., a discrete point 401a) can be established on the first large arm 401; b discrete points (i.e., a discrete point 4021, a discrete point 4022, ..., discrete point 402b) can be established on the first middle arm 402; c discrete points (i.e., a discrete point 4031, a discrete point 4032, ..., a discrete point 403c) can be established on the first small arm 403; d discrete points (i.e., a discrete point 4041, a discrete point 4042, ..., a discrete point 404d) can be established on the first surgical instrument 404. Where, the a, b, c, and d are all natural numbers. Similarly, the mechanical arm body of the second tool arm 41 can include a second large arm, a second middle arm, and a second small arm which are sequentially connected by joints. The distal end of the mechanical arm body of the second tool arm 41 (i.e., the second small arm) can be configured to mount a detection instrument, such as an endoscope. The discrete points can be similarly arranged on the second large arm, the second middle arm, the second small arm, and the detection instrument. The mechanical arm body of the third tool arm 42 can also include a third large arm, a third middle arm, and a third small arm which are sequentially connected by joints. The distal end of the mechanical arm body of the third tool arm 42 (i.e., the third small arm) can be configured to mount a second surgical instrument. The discrete points can be similarly arranged on the third large arm, the third middle arm, the third small arm, and the second surgical instrument. While the discrete points are arranged, the discrete point information, such as the first relative position information between the discrete point and the corresponding joint and the discrete point gain coefficient of each discrete point, can be acquired. "A joint corresponding to a discrete point" may refer to a joint connected to a proximal end of a connecting rod (or a medical instrument) where the discrete point is on.

Further, as shown in FIGS. 3-5, since the position sensor 10 is provided on the joint of each mechanical arm, the coordinate information (that is, the second coordinate information) of each joint in the global coordinate system can be acquired according to each position sensor 10 and each parameter of a kinematic model stored in the memory 12 (for example, a parameter such as a length of the connecting rod in the D-H parameter method). Furthermore, by combining the position relationship of each discrete point relative to the corresponding joint thereof (i.e. first relative position information) obtained when arranging the discrete points, the coordinate information of each discrete point in the global coordinate system (i.e. the first coordinate information) can be solved.

It should be noted that each of the exemplary embodiments in the present disclosure has no particular limitation on the relationship between the first relative distance and the first interaction force. A value range of the first relative distance is $(0, +\infty)$, so long as the first interaction force is negatively correlated with the first relative distance within at least one distance interval in the value range $(0, +\infty)$. For example, the value range $(0, +\infty)$ includes only one distance interval, that is, the first interaction force is negatively correlated with the first relative distance in the distance interval $(0, +\infty)$. Alternatively, the entire value range $(0, +\infty)$ includes a plurality of distance intervals, the first interaction force and the first relative distance may have different correspondences in different distance intervals. The corresponding relationship between the first relative distance and the first interaction force will be further detailed with reference to FIG. 6.

FIG. 6 is a schematic curve diagram of a relationship between a first relative distance and a first interaction force according to an optional embodiment. In the specific embodiment shown in FIG. 6, the vertical coordinate F represents a first interaction force and the horizontal coordinate $L$ represents a first relative distance between two discrete points respectively located on different mechanical arms. The memory 12 stores three distance intervals, specifically including a first distance interval $(0, L_{min} + R_m + R_n]$, a second distance interval $(L_{min} + R_m + R_n, L_{max} + R_m + R_n)$, and a third distance interval $[L_{max} + R_m + R_n, +\infty)$. Where, $R_m$, $R_n$ are maximum radiuses of sections of the mechanical arm at the positions where the two discrete points are located, and are obtained according to the size of the mechanical arm on which the two discrete points are located; $L_{max}$ is a preset collision warning distance, and $L_{min}$ is a preset minimum safe distance.

In an optional embodiment, the first interaction force F is a preset maximum interaction force $F_{max}$ in the range of the first distance interval $(0, L_{min} + R_m + R_n]$. In the second distance interval $(L_{min} + R_m + R_n, L_{max} + R_m + R_n)$, the relationship between the first interaction force and the first relative distance between the two discrete points is a function which is a continuous function in the second distance interval, and a first derivative of the function is less than zero and a second derivative of the function is greater than zero. In the third distance interval $[L_{max} + R_m + R_n, +\infty)$, the first interaction force $F$ is zero. In other words, in the present embodiment, the first interaction force $F_{m,n}$ between any two discrete points m, n located on different mechanical arms and the first relative distance $L$ between the two discrete points may satisfy the following relationship:

$$
F_{m,n} = \begin{cases} 0 & L \geq L_{max} + R_m + R_n \\ f(\mathrm{L}) & L_{min} + R_m + R_n < L < L_{max} + R_m + R_n \\ F_{max} & L \leq L_{min} + R_m + R_n \end{cases}
$$

Where, $F_{m,n}$ is the first interaction force of the discrete point $m$ with respect to the discrete point $n$, and the direction of $F_{m,n}$ is from the discrete point $m$ to the discrete point $n$, and a value of $F_{m,n}$ is equal to that of $F_{n,m}$, but a direction of $F_{m,n}$ is opposite to that of $F_{n,m}$; $L$ is a first relative distance between the discrete point $m$ and the discrete point $n$; $R_m$ is a maximum radius of a section of the mechanical arm at the discrete point $m$ which is on the mechanical arm; and $R_n$ is a maximum radius of a section of the mechanical arm at the discrete point $n$ which is on the mechanical arm; $F_{max}$ represents the maximum force; $f(\mathrm{L})$ is a continuous function within the second distance interval $(L_{min} + R_m + R_n, L_{max} + R_m + R_n)$, and the first derivative of the function is less than zero and the second derivative is greater than zero. Therefore, as the first relative distance $L$ increases, the value of the first interaction force $F_{m,n}$ obtained by calculating according to the above-mentioned function decreases at a faster speed.

In an optional embodiment, the function $f(\mathrm{L})$ can be provided as follows:

$$
f(L) = k(\frac{1}{L} - \frac{1}{L_{max}})(\frac{1}{L^2})L',
$$

Where, $k$ is a distance-force gain coefficient, $L$ can be a first relative distance, and $L'$ can be a derivative of $L$ with respect to time, and configured to represent a rate of change of the first relative distance between the two discrete points $m$, $n$ with respect to time, specifically, which can be obtained by calculating a difference value between a current first relative distance between the two discrete points $m$ and $n$ and a first relative distance between the two discrete points $m$ and $n$ in a previous control period (i.e., collision detection interval time), and dividing the difference value by the control period. The control period can be, for example, 0.5 ms, i.e., a collision detection is performed every 0.5 ms.

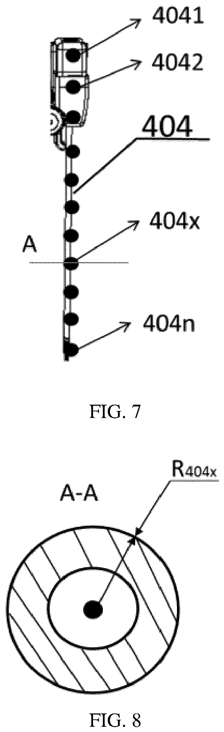

FIG. 7 is a schematic diagram of arranging discrete points for a surgical instrument in a teleoperation medical robot according to an optional embodiment, and Fig. 8 is a schematic diagram illustrating a section A-A at a discrete point 404x along a line A shown in FIG. 7. The first mechanical arm 40 is taken as an example to describe with reference to FIGS. 3-8. In this embodiment, a maximum radius R404x of a section at any discrete point 404x (x=1, 2, ..., a) on the first surgical instrument 404 of the mechanical arm can be obtained in advance according to the information during the instrument design of the first surgical instrument 404. Similarly, the maximum radius R of a section at a discrete point on the remaining mechanical arms can be known in advance. The section herein is understood as a cross section, i.e., a cross section perpendicular to an axis of the surgical instrument. Thus, the $R_m$, $R_n$ can be obtained.

In the present embodiment, the operator can preoperatively preset a plurality of distance intervals and a corresponding relationship between the first relative distance and the first interaction force within each distance interval by using the processor 11, and stores in the memory 12 for subsequent retrieval. Apparently, other devices can also be utilized to preset the distance interval and the corresponding relationship between the first relative distance and the first interaction force in each distance interval when the device is initialized or leaves the factory, and pre-store in the memory 12. Further, the processor 11 can acquire an interaction force (i.e., the first interaction force) corresponding to each discrete point and a discrete point on other mechanical arms based on the corresponding relationship between the first relative distance and the first interaction force stored in the memory 12, and obtain the resultant force of all the first interaction forces according to the interaction forces, and then obtain the Cartesian force of each mechanical arm based on the resultant force of all the first interaction forces corresponding to each discrete point. Alternatively, the system for preventing a collision between mechanical arms further includes an input device. The operator can temporarily set a plurality of distance intervals and the corresponding relationship between the first relative distance and the first interaction force within each distance interval through the input device during the surgery. In this case, the processor 11 can also directly acquire the relationship between the first relative distance and the first interaction force.

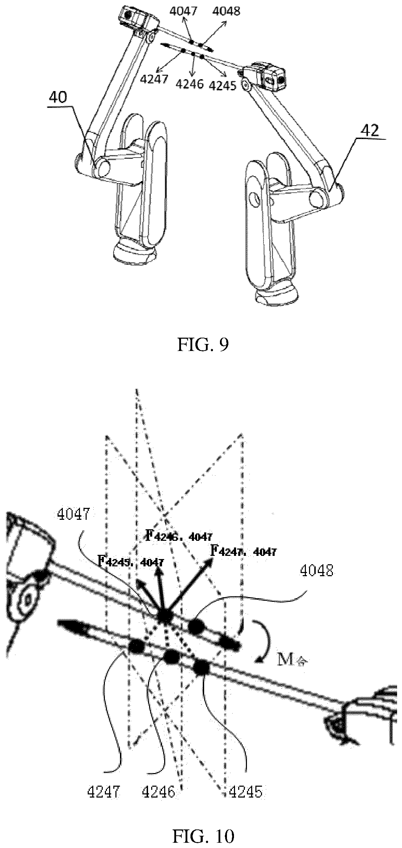

FIG. 9 is a schematic diagram illustrating a pose of a mechanical arm of a teleoperation medical robot according to an optional embodiment (the second mechanical arm 41 is omitted in order to facilitate the observation); and FIG. 10 is a schematic diagram illustrating a force on each discrete point in FIG. 9. As shown in Fig. 9, surgical instruments can be mounted on the distal ends of the mechanical arm bodies of the first mechanical arm 40 and the third mechanical arm 42, and the surgical instruments can be driven to move around a fixed point. For each discrete point of the surgical instrument on the first mechanical arm 40, the first interaction force of all discrete points on the third mechanical arm 42 with respect to any discrete point of the surgical instrument mounted on the first mechanical arm 40, and the first interaction force of all discrete points on the second mechanical arm 41 with respect to any discrete point of the surgical instrument on the first mechanical arm 40 can be obtained through the corresponding relationship between the first relative distance between the different discrete points and the first interaction force. Further, the magnitude and direction of the resultant force of all the first interaction forces received by each discrete point on the surgical instrument of the first mechanical arm 40 are calculated, and then a resultant torque generated by the first interaction forces of all discrete points on the surgical instrument with respect to the joint connecting the surgical instrument with the mechanical arm body is obtained according to the resultant force of the first interaction forces corresponding to each discrete point which corresponds to a joint connecting the surgical instrument with the mechanical arm body (i.e., each discrete point on the surgical instrument), and the first relative position of each discrete point with respect to the joint, and the gain coefficient $g_i$ of each discrete point. Further, the processor 11 calculates the resultant torque corresponding to all joints on the first mechanical arm 40, and can further obtain the Cartesian force of the first mechanical arm 40 according to a force Jacobian matrix of the first mechanical arm 40 in a current configuration at this time.

Specifically, when the first mechanical arm 40 and third mechanical arm 42 are in the configurations shown in FIG. 10, the coordinates of all discrete points in global coordinate system can be determined according to parameters of the position sensor and the first relative position of each joint on the first mechanical arm 40 and third mechanical arm 42. In the present embodiment, by determining the first relative distance between the discrete points respectively on the two mechanical arms (i.e., the first mechanical arm 40 and the third mechanical arm 42), it is found that, except that each first interaction force between the discrete point 4047 on the first mechanical arm 40 and each of the discrete points 4245, 4246, 4247 on the third mechanical arm 42, and each first interaction force between the discrete point 4048 on the first mechanical arm 40 and each of the discrete points 4245, 4246, 4247 on the third mechanical arm 42 in the figure are not zero, first interaction forces between the remaining discrete points are zero (that is, the distance is farther, and the first relative distance is greater than $L_{max} + R_m + R_n$). Thus, the resultant force $F_{4047}$ of the first interaction forces corresponding to the discrete point 4047 on the first mechanical arm is obtained according to the first interaction forces $F_{4245,4047}$, $F_{4246,4047}$, $F_{4247,4047}$ of the discrete points 4245, 4246, and 4247 on the third mechanical arm 42 with respect to the discrete point 4047 on the first mechanical arm 40, , and then a torque of the discrete point 4047 on the first mechanical arm with respect to the rotational joint is obtained as $M_{4047}$. Similarly, the torque of the discrete point 4048 on the first mechanical arm with respect to the rotational joint is $M_{4048}$, so that a resultant torque corresponding to a joint between the first mechanical arm body and the first surgical instrument satisfies $M_r = M_{4047} * g_{4047} + M_{4048} * g_{4048}$. By analogy, after a resultant torque corresponding to each joint of the first mechanical arm is obtained, the resultant torque of each joint of the first mechanical arm 40 is converted to the Cartesian force at the distal end of the first tool arm in a Cartesian space according to the force Jacobian matrix in the current configuration of the first mechanical arm 40.

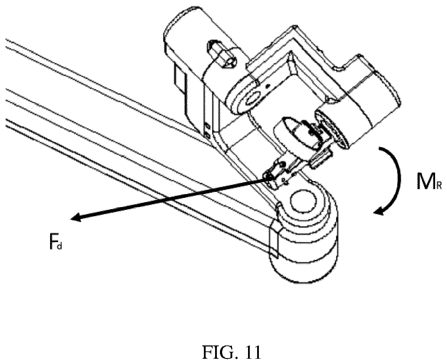

FIG. 11 is a schematic diagram illustrating a force on a master manipulator in a teleoperation medical robot according to an optional embodiment. As shown in FIGS. 1 to 11, since the first mechanical arm 40 is controlled by the first master manipulator 31 at the physician side. In order to make the operator perceive the Cartesian force, the Cartesian force $F_d$ of the first master control arm 31 can be obtained according to the obtained Cartesian force of the first mechanical arm 40, and the torque of each joint of the first master control arm 31 is obtained according to the force Jacobian matrix of the first master manipulator 31, and then a resistance torque $M_R$ (with the same magnitude of the torque of each joint of the first master control arm 31, but opposite direction to the torque of each joint of the first master control arm 31) output by a motor and configured to control the movement of each joint of the first master control arm 31 is obtained to feed back to the operator.

Specifically, the control end can also be configured to scale the Cartesian force of the first mechanical arm 40 to obtain the Cartesian force $F_d$ of the first master manipulator 30 at the physician side. Then, the control end can obtain the resistance torque $M_R$ output by the motor and configured to control the movement of each joint of the first master manipulator 30 according to the force Jacobian matrix of the first master manipulator 30. Similarly, after the torque of each joint of the second master control arm 31 is obtained, the control end can transmit the torque for each joint of the first master manipulator 30 and the second master manipulator 31 to the motor of the master manipulator to output a resistance torque with the same magnitude with but opposite direction to the torque, so that the operator encounters a corresponding resistance during the operation, and accordingly perceives a Cartesian force of each mechanical arm, thereby achieving the purpose of accurately controlling and effectively avoiding the collision between the mechanical arms.

In another optional embodiment, the system for preventing the collision further includes a display device which is configured to display a Cartesian force corresponding to a distal end of each mechanical arm in an information representation such as text, graphics, images, etc., so that the Cartesian force is perceived by the operator.

In another optional embodiment, as shown in FIG. 4, the system for preventing the collision can further include an alarm 13 in communication with the processor 11, i.e., when a first relative distance $L$ between any two discrete points on different mechanical arms is less than a preset distance threshold, and/or a Cartesian force of any one of the mechanical arms exceeds a preset force threshold, the alarm 13 is triggered to send out a warning message in a manner such as an alarm bell, an indicator lamp, or the like to alert of an imminent collision. For example, the distance threshold can be equal to $L_{min} + R_m + R_n$. For another example, the distance threshold can further include a first distance threshold $L_{min} + R_m + R_n$ and a second distance threshold $L_{max} + R_m + R_n$ ; and when a first relative distance $L$ between any two discrete points is less than the first distance threshold, the alarm sends out first alarm information (e.g., a red light signal is sent out); when the first relative distance $L$ between any two discrete points is greater than or equal to a first distance threshold $L_{min} + R_m + R_n$ and less than a second distance threshold $L_{max} + R_m + R_n$, the alarm sends out second alarm information (e.g., a yellow light signal is sent out); when the first relative distance $L$ between any two discrete points is greater than the second distance threshold $L_{max} + R_m + R_n$, the alarm is dormant or sends out normal operation information (e.g., a green light signal is sent out).

In the above-mentioned embodiments, the system for preventing the collision can be used for detecting the collision between two or more mechanical arms, in which the mechanical arm can include a mounted medical instrument. It will be appreciated by those skilled in the art that the system and method for preventing the collision in the embodiments of the present disclosure can be used for detecting the collision between mechanical arm bodies, collision between the mechanical arm body and the medical instrument, and/or collision between the medical instruments, and the like.

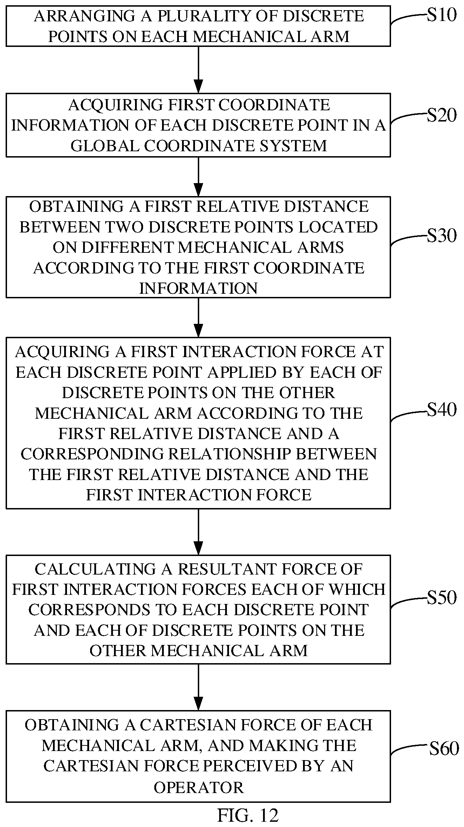

FIG. 12 is a flow chart showing a method for preventing a collision between mechanical arms according to an optional embodiment. As shown in FIG. 12, in an optional embodiment, the present disclosure further provides a mechanical arm anti-collision detection method, which can be applied to a medical robot, such as a teleoperation medical robot as shown in FIGS. 1 to 11. The medical robot can have at least two mechanical arms (e.g., two, three, four, etc.), and each mechanical arm can include a mechanical arm body; and the mechanical arm body can include a plurality of joints and a plurality of connecting rods connected by the plurality of joints. For example, the joints on each joint arm body are connected to the connecting rods in sequence. Meanwhile, a position sensor for obtaining joint position information is provided on each joint. At least a part of the mechanical arm can also include a medical instrument mounted on the distal end of the mechanical arm body, such as a surgical instrument or a detection instrument. Since when the mechanical arms of the medical robot operate in coordination, the mechanical arms (including but not limited to between the mechanical arm bodies, between the medical instruments, and between the mechanical arm body and the medical instrument) are easily interfered and collided with each other due to a small interval, the method of the present example is improved inventively with respect to the above defects, and can include the following steps.

At step S10, a plurality of discrete points are provided on each mechanical arm.

Specifically, the discrete points are arranged on each mechanical arm according to factors such as force collision detection sensitivity, surgical operation requirements, and the size of each component of the mechanical arm. For example, a plurality of discrete points can be arranged on each mechanical arm body. In order to improve the force collision detection sensitivity, the various discrete points can be uniformly arranged on each connecting rod, or the discrete points can be distributed in a region where collision is relatively easy to occur according to information such as the shape and size of each connecting rod, or each of the discrete points can be uniformly distributed on the mechanical arm. The size information may specifically include various three-dimensional geometric parameters such as lengths, shapes, and volumes of each joint and connecting rod on the mechanical arm.

In an optional embodiment, the first relative position information of each discrete point with respect to the corresponding joint and the parameter information such as a discrete point gain coefficient of each discrete point can be also continuously obtained.

It should be noted that when the mechanical arm in the embodiment of the present disclosure includes the medical instrument mounted at the distal end of the mechanical arm body, discrete points can be arranged on the mechanical arm body and/or the medical instrument, that is, the medical instrument can be considered as one connecting rod of the mechanical arm body, so that the anti-collision detection can be performed on the mechanical arm body and the mounted medical instrument as a whole.

At step S20, first coordinate information of each discrete point in a global coordinate system is acquired.

Specifically, the second coordinate information of each discrete point in the global coordinate system can be acquired according to joint position information obtained by the position sensor and the preset kinematics model; and the first coordinate information of each discrete point in the global coordinate system is obtained according to the second coordinate information and the first relative position information.

In an optional embodiment, the plurality of discrete points can be arranged and the first relative position information can be obtained after the second coordinate information is obtained; and then the first coordinate information is obtained according to the second coordinate information and the first relative position information.

At step S30, a first relative distance between two discrete points located on different mechanical arms is obtained according to the first coordinate information.

Specifically, the first relative distance between discrete points respectively located on different mechanical arms can be obtained according to the first coordinate information. That is, the distance between any two discrete points respectively located on different mechanical arms is calculated according to the first coordinate information of the two discrete points, to obtain the first relative distance between any two discrete points respectively located on different mechanical arms.

At step S40, a first interaction force corresponding to each discrete point and each discrete point on the other mechanical arm is acquired according to the first relative distance and a corresponding relationship between the first relative distance and the first interaction force.

In the exemplary embodiments of the present disclosure, there is no specific limitation on the relationship between the first relative distance and the first interaction force. A value range of the first relative distance is $(0, +\infty)$ as long as the first interaction force is negatively correlated with the first relative distance within at least one distance interval in the value range $(0, +\infty)$.

In an optional embodiment, when the value range of the first relative distance includes only one distance interval, i.e., the first interaction force is negatively correlated with the first relative distance in the distance interval $(0, +\infty)$.

In another optional embodiment, the value range of the first relative distance can include a plurality of different distance intervals, and the corresponding relationship between the first relative distance and the first interaction force in each distance interval is at least partially different.

For example, the value range includes a first distance interval $(0, L_{min} + R_m + R_n]$, a second distance interval $(L_{min} + R_m + R_n, L_{max} + R_m + R_n)$, and a third distance interval $[L_{max} + R_m + R_n, +\infty)$. Here, $R_m$ is cross-sectional maximum radius of the mechanical arm (e.g., the first mechanical arm 40) at the discrete point $m$, in which the discrete point $m$ is located on the mechanical arm; $R_n$ is cross-sectional maximum radius of the mechanical arm (e.g., the second mechanical arm 41) at the discrete point $n$, in which the discrete point $n$ is located on the mechanical arm; and $L_{max}$ is a preset collision warning distance; $L_{min}$ is a preset minimum safe distance. Then, the first interaction force F is a preset maximum interaction force $F_{max}$ in the first distance interval $(0, L_{min} + R_m + R_n]$. The corresponding relationship between the first interaction force and the first relative distance between the two discrete points is a function within the second distance interval $(L_{min} + R_m + R_n, L_{max} + R_m + R_n)$; the function is a continuous function in the second distance interval, and the first derivative of the function is less than zero, and the second derivative of the function is greater than zero. The first interaction force $F$ is zero in the third distance interval $[L_{max} + R_m + R_n, +\infty)$. The corresponding relationship between the first relative distance and the first interaction force can be represented by the following formula:

$$
F_{m,n} = \begin{cases} 0 & L \geq L_{\max} + R_m + R_n \\ f(\mathrm{L}) & L_{\min} + R_m + R_n < L < L_{\max} + R_m + R_n \\ F_{\max} & L \leq L_{\min} + R_m + R_n \end{cases}
$$

In the formula, $F_{m,n}$ is the first interaction force of the discrete point $m$ with respect to the discrete point $n$, with a direction from the discrete point $m$ to the discrete point $n$, and accordingly, $F_{n,m}$ is the first interaction force of the discrete point $n$ with respect to the discrete point $m$ with a direction from the discrete point $n$ to the discrete point $m$, i.e., $F_{m,n}$ and $F_{n,m}$ have the same value but opposite directions.

In another optional embodiment, the function which is continuous in the second distance interval can be:

$$f(L) = k(\frac{1}{L} - \frac{1}{L_{\max}}) \ (\frac{1}{L^2})L'$$

Where, $k$ is a distance-force gain coefficient and $L$ is the first relative distance between the discrete points; $L'$ is a derivative of $L$ with respect to time, and is configured to characterize a rate of change of the first relative distance between the two discrete points with respect to time, which can be obtained by calculating a difference value between a current first relative distance between any two discrete points and a first relative distance between the two discrete points in the previous control period (i.e., collision detection interval time), and dividing the difference value by the control period.

At step S50, a resultant force of first interaction forces each of which corresponds to each discrete point and each discrete point on the other mechanical arm is calculated.

Specifically, for any discrete point, a first interaction force corresponding to the discrete point and each of all discrete points located on the other mechanical arm is acquired, and a vector summation is performed on all first interraction forces to obtain a resultant force of all the first interaction forces corresponding to the discrete point.

At step S60, a Cartesian force of each mechanical arm is acquired and the Cartesian force is perceived by the operator.

Specifically, a torque of a joint corresponding to each discrete point pair is obtained according to the resultant force of all first interaction forces corresponding to each discrete point and first relative position information of each discrete point with respect to a corresponding joint; and a resultant torque corresponding to each joint is obtained by combining the discrete point gain coefficients $g_i$; and then, the Cartesian force of each mechanical arm is obtained according to the resultant torque corresponding to each joint and a force Jacobian matrix of the mechanical arm.

In the present embodiment, there is no specifically limit to the method by which the Cartesian force of each mechanical arm is perceived by the operator. In a specific embodiment, the Cartesian force of the master manipulator 31 controlling the movement of the mechanical arm can be obtained according to the acquired Cartesian force of the mechanical arm, and then the torque of each joint of the master manipulator 31 can be obtained by means of such as the force Jacobian matrix of the mechanical arm; and the motor is subsequently controlled to output a resistance torque with the same magnitude as but opposite direction to the torque of each joint of the master manipulator 31, so that the operator's hand can perceive the Cartesian force of the mechanical arm.

In another specific embodiment, the Cartesian force can also be displayed through a display device in an information representation such as text, graphics, images, etc., so that the operator can perceive the Cartesian force through eyes.

In another optional embodiment, when the distance between any two discrete points respectively located on different mechanical arms is less than a preset distance threshold, or the obtained Cartesian force is greater than a predetermined alarm force, a warning message such as sound, light, electricity, vibration, or the like is triggered. For example, the distance threshold can be set to $L_{\min} + R_m + R_n$. For another example, the distance threshold can include a first distance threshold $L_{\min} + R_m + R_n$ and a second distance threshold $L_{\max} + R_m + R_n$; and when the first relative distance $L$ between the two discrete points is less than the first distance threshold $L_{\min} + R_m + R_n$, the alarm sends out first alarm information (e.g., sending out a red light signal); when the first relative distance $L$ between the two discrete points is greater than or equal to the first distance threshold $L_{\min} + R_m + R_n$ and less than the second distance threshold $L_{\max} + R_m + R_n$, the alarm sends out second alarm information (e.g., sending out a yellow light signal); when the first relative distance $L$ between the two discrete points is greater than the second distance threshold $L_{\max} + R_m + R_n$, the alarm is dormant or sends out normal operation information (e.g., sending out a green light signal).

It should be appreciated that although the steps in the flow chart of FIG. 12 are shown in sequence as indicated by the arrows, these steps are not definitely performed in sequence as indicated by the arrows. Unless expressly stated herein, these steps are not performed in a strict order and may be performed in other orders. Further, at least part of the steps in FIG. 12 can include a plurality of sub-steps or a plurality of stages, which are not definitely performed at the same time, but may be performed at different time; and the sub-steps or stages may not be definitely performed sequentially, but can be executed in turns or alternately with at least part of other steps or sub-steps or stages of other steps.

Persons of ordinary skill in the art understand that all or part of the processes in the methods of the above-mentioned embodiments may be implemented by a computer program instructing a relevant hardware. The computer program may be stored in a non-transitory computer-readable storage medium. When the computer program is executed, flows in the embodiments of the method can be included. Any reference to the memory, storage, database, or other media used in various embodiments provided herein may include a non-transitory and/or transitory memory. The non-transitory memory may include a read only memory (ROM), a programmable ROM (PROM), an electrically programmable ROM (EPROM), an electrically erasable programmable ROM (EEPROM), or a flash memory. The transitory memory may include a random access memory (RAM) or an external cache memory. By way of illustration and not limitation, RAM is available in a variety of forms such as static RAM (SRAM), dynamic RAM (DRAM), synchronous DRAM (SDRAM), double data rate SDRAM (DDRSDRAM), enhanced SDRAM (ESDRAM), Synchlink DRAM (SLDRAM), Memory Bus (Rambus) Direct RAM (RDRAM), Direct Memory Bus Dynamic RAM (DRDRAM), Memory Bus Dynamic RAM (RDRAM) etc.

The above-mentioned various technical features involved in the respective embodiments can be combined arbitrarily, for brevity, not all possible combinations of the technical features in the above-mentioned embodiments are described. However, as long as there is no conflict in the combinations of the technical features, the combinations of the technical features shall be considered as the scope of the disclosure.

The above-mentioned embodiments are merely some exemplary embodiments of the present disclosure. The descriptions are more specific and detailed, and are not intended to limit the protection scope of the present disclosure. It should be pointed out that those of ordinary skill in the art can make several modifications and improvements without departing from the concept of the disclosure, and these all fall within the scope of protection of the disclosure. Therefore, the protection scope of the present disclosure shall be subject to the appended claims.

**Claims**

1. A method for preventing a collision between mechanical arms, applied to a medical robot, the medical robot comprising at least two mechanical arms, a mechanical arm of the at least two mechanical arms comprising a mechanical arm body, the mechanical arm body comprising a plurality of joints, the method comprising:

   arranging a plurality of discrete points on each mechanical arm;
   acquiring first coordinate information of each discrete point in a global coordinate system;
   obtaining a first relative distance between two discrete points located on different mechanical arms according to the first coordinate information;
   acquiring a first interaction force corresponding to each discrete point and each of discrete points on the other mechanical arm according to the first relative distance and a corresponding relationship between the first relative distance and the first interaction force; and
   obtaining a Cartesian force of each mechanical arm according to a resultant force of first interaction forces each of which corresponds to each discrete point and each of discrete points on the other mechanical arm, and making the Cartesian force perceived by an operator.

2. The method according to claim 1, wherein at least a part of the mechanical arms further comprises a medical instrument mounted on a distal end of the mechanical arm body, the arranging the plurality of discrete points on each mechanical arm comprises arranging the discrete points on a medical instrument and/or the mechanical arm body.

3. The method according to claim 1 or 2, wherein the arranging the plurality of discrete points on each mechanical arm comprises:
   acquiring first relative position information of a discrete point with respect to a corresponding joint.

4. The method according to claim 3, wherein the mechanical arm further comprises a position sensor configured to acquire joint position information, the acquiring the first coordinate information of each discrete point in the global coordinate system comprises:

   obtaining the joint position information of the joint by the position sensor, and obtaining second coordinate information of each joint in the global coordinate system according to the joint position information and a kinematic model; and
   obtaining the first coordinate information of the discrete point in the global coordinate system according to the second coordinate information and the first relative position information.

5. The method according to claim 1 or 2, wherein the acquiring the first interaction force corresponding to each discrete point and each of discrete points on the other mechanical arm according to the first relative distance and the corresponding relationship between the first relative distance and the first interaction force comprises:

   setting a plurality of distance intervals in a value range of the first relative distance, wherein the corresponding relationship corresponding to at least one of the distance intervals is different from corresponding relationships corresponding to other distance intervals; and

obtaining the first interaction force corresponding to each discrete point and each of discrete points on the other mechanical arm according to the first relative distance between the discrete points and the corresponding relationship corresponding to a distance interval which the first relative distance is in.

6. The method according to claim 5, wherein

the plurality of distance intervals includes a first distance interval $(0, L_{min} + R_m + R_n]$, a second distance interval $(L_{min} + R_m + R_n, L_{max} + R_m + R_n)$, and a third distance interval $[L_{max} + R_m + R_n, +\infty)$; and
the first interaction force F is a preset maximum interaction force $F_{max}$ in the first distance interval $(0, L_{min} + R_m + R_n]$; a relationship between the first interaction force and the first relative distance is a function in the second distance interval $(L_{min} + R_m + R_n, L_{max} + R_m + R_n)$, the function is a continuous function in the second distance interval, a first derivative of the function is less than zero, and a second derivative of the function is greater than zero; the first interaction force F is equal to zero in the third distance interval $[L_{max} + R_m + R_n, +\infty)$;
wherein discrete points $m$ and $n$ are two discrete points on different mechanical arms, $R_m$ is a cross-sectional maximum radius of a mechanical arm at the discrete point $m$, with the discrete point $m$ located on the mechanical arm; $R_n$ is a cross-sectional maximum radius of a mechanical arm at the discrete point $n$, with the discrete point $n$ located on the mechanical arm; $L_{max}$ is a preset collision warning distance of the first relative distance, and $L_{min}$ is a preset minimum safe distance of the first relative distance.

7. The method according to claim 6, wherein the function is:

$$f(L) = k(\frac{1}{L} - \frac{1}{L_{max}})(\frac{1}{L^2})L',$$

$k$ is a distance-force gain coefficient, $L$ is the first relative distance, and $L'$ is a derivative of $L$ with respect to time.

8. The method according to claim 3, wherein the arranging the plurality of discrete points on each mechanical arm further comprises:

acquiring a discrete point gain coefficient of each discrete point;
wherein the obtaining the Cartesian force of each mechanical arm according to the resultant force of the first interaction forces each of which corresponds to each discrete point and each of discrete points on the other mechanical arm comprises:

obtaining a resultant force of all first interaction forces corresponding to each discrete point;
obtaining a torque corresponding to each discrete point with respect to a corresponding joint according to the resultant force of all the first interaction forces corresponding to each discrete point and the first relative position information of each discrete point with respect to a corresponding joint, and obtaining a resultant torque corresponding to each joint according to a discrete point gain coefficient of each discrete point; and obtaining the Cartesian force of the mechanical arm according to the resultant torque corresponding to each joint and a force Jacobian matrix of the mechanical arm.

9. The method according to claim 1 or 2, wherein the making the Cartesian force perceived by the operator comprises: displaying the Cartesian force by a display device.

10. The method according to claim 1 or 2, wherein the mechanical arm is connected to a master manipulator configured to control a movement of the mechanical arm, the making the Cartesian force perceived by the operator comprises:

obtaining a Cartesian force of the master manipulator configured to control the movement of the mechanical arm according to the Cartesian force of the mechanical arm;
obtaining a torque corresponding to each joint of the master manipulator according to a force Jacobian matrix and the Cartesian force of the master manipulator; and
causing a motor configured to control a movement of a joint of the master manipulator to correspondingly output a resistance torque having a same magnitude as but an opposite direction to the torque corresponding to each joint of the master manipulator.

11. A system for preventing a collision between mechanical arms, applied to a medical robot, the medical robot comprising at least two mechanical arms, a mechanical arm comprising a mechanical arm body, the mechanical arm body comprising a plurality of joints, the system comprising:

a discrete point arranging unit configured to arrange a discrete point on the mechanical arm;
a memory configured to store instructions;
a processor in communication with the discrete point arranging unit and the memory, respectively;
wherein the instructions stored in the memory, when executed by the processor, perform the steps of:

acquiring first coordinate information of each discrete point in a global coordinate system;
obtaining a first relative distance between two discrete points on different mechanical arms according to the first coordinate information;
acquiring a first interaction force corresponding to each discrete point and each of discrete points on the other mechanical arm according to the first relative distance and a corresponding relationship between the first relative distance and the first interaction force; and
obtaining a Cartesian force of each mechanical arm according to a resultant force of first interaction forces each of which corresponds to each discrete point and each of discrete points on the other mechanical arm, and making the Cartesian force perceived by an operator.

12. The system according to claim 11, wherein at least a part of the mechanical arms further comprises a medical instrument mounted on a distal end of the mechanical arm body, and the discrete point is arranged on the medical instrument and/or the mechanical arm body.

13. The system according to claim 11, wherein,

the mechanical arm further comprises a position sensor configured to acquire joint position information, the position sensor being in communication with the processor;
the discrete point arranging unit is further configured to acquire first relative position information of the discrete point with respect to a corresponding joint;
the instructions stored on the memory, when executed by the processor, further perform the steps of:

acquiring, by the position sensor, joint position information of the joint, and acquiring second coordinate information of each joint in the global coordinate system according to the joint position information and a kinematic model; and
acquiring the first coordinate information of each discrete point in the global coordinate system according to the second coordinate information of each joint in the global coordinate system and the first relative position information.

14. The system according to claim 11, wherein,

the corresponding relationship between the first relative distance and the first interaction force is pre-stored in the memory;
or,
the system further comprises an input device configured to acquire the corresponding relationship between the first relative distance and the first interaction force during a surgery, to directly be executed by the processor or stored in the memory.

15. The system according to any one of claims 11 to 13, wherein a value range of the first relative distance includes a plurality of distance intervals, and the corresponding relationship corresponding to at least one of the distance intervals is different from corresponding relationships corresponding to other distance intervals;
wherein the instructions stored on the memory, when executed by the processor, further perform the steps of:
obtaining a first interaction force corresponding to each discrete point and each of discrete points on the other mechanical arm according to the first relative distance of each discrete point and a corresponding relationship corresponding to a distance interval which the first relative distance is in.

16. The system according to claim 15, wherein

the preset plurality of distance intervals comprise a first distance interval $(0, L_{min} + R_m + R_n]$, a second distance

interval $(L_{min} + R_m + R_n, L_{max} + R_m + R_n)$, and a third distance interval $[L_{max} + R_m + R_n, +\infty)$; and the first interaction force F is a preset maximum interaction force $F_{max}$ in the first distance interval $(0, L_{min} + R_m + R_n]$; a relationship between the first interaction force and the first relative distance is a function in the second distance interval $(L_{min} + R_m + R_n, L_{max} + R_m + R_n)$, the function is a continuous function in the second distance interval, and a first derivative of the function is less than zero, and a second derivative of the function is greater than zero; the first interaction force F is zero in the third distance interval $[L_{max} + R_m + R_n, +\infty)$;

wherein discrete points $m$ and $n$ are two discrete points on different mechanical arms, $R_m$ is a cross-sectional maximum radius of a mechanical arm at the discrete point $m$, with the discrete point $m$ located on the mechanical arm; $R_n$ is a cross-sectional maximum radius of a mechanical arm at the discrete point $n$, with the discrete point $n$ located on the mechanical arm; $L_{max}$ is a preset collision warning distance of the first relative distance, and $L_{min}$ is a preset minimum safe distance of the first relative distance.

**17.** The system according to claim 16, wherein the function is:

$$f(L) = k(\frac{1}{L} - \frac{1}{L_{max}})(\frac{1}{L^2})L',$$

$k$ is a distance-force gain coefficient, $L$ is the first relative distance, and $L'$ is a derivative of L with respect to time.

**18.** The system according to claim 11 or 12, wherein the discrete point arranging unit is further configured to obtain a discrete point gain coefficient of each discrete point;
the instructions stored in the memory, when executed by the processor, further perform steps of:

obtaining a resultant force of first interaction forces each of which corresponds to each discrete point and each of discrete points on the other mechanical arm;
obtaining a torque corresponding to each discrete point with respect to a corresponding joint according to the resultant force of the first interaction forces corresponding to each discrete point and the first relative position information of each discrete point with respect to a corresponding joint, and obtaining a resultant torque corresponding to each joint according to the torque corresponding to the discrete point with respect to the corresponding joint and the discrete point gain coefficient of each discrete point; and
obtaining a Cartesian force of the mechanical arm according to the resultant torque corresponding to each joint and a force Jacobian matrix of the mechanical arm.

**19.** The system according to claim 11, further comprising an alarm connected to the processor, wherein the instructions stored on the memory, when executed by the processor, further perform steps of:
when a first relative distance of any discrete point on the mechanical arm is less than or equal to a preset warning distance, and/or when the Cartesian force of the mechanical arm is greater than a preset force threshold, triggering the alarm to send out a warning message.

**20.** The system according to claim 11 or 12, further comprising:
a display device, wherein the processor is further configured to display the Cartesian force through the display device.

**21.** A medical robot, comprising:

at least two mechanical arms, a mechanical arm comprising a mechanical arm body, the mechanical arm body comprising a plurality of joints; and
the system for preventing the collision between mechanical arms according to any one of claims 11 to 20.

**22.** The medical robot according to claim 21, further comprising a physician side, a patient side, and a control end;

wherein the physician side, the patient side, and the system for preventing the collision between mechanical arms are respectively in communication with the control end; the mechanical arm is located at the patient side; the physician side comprises a master manipulator, the master manipulator comprises a plurality of joints and a motor driving the joint to move, the master manipulator is configured to control a movement of the mechanical arm; the control end is configured to set a Cartesian force of the master manipulator according to a Cartesian force of the mechanical arm obtained from the system for preventing the collision between the mechanical arms;

and

the control end is further configured to obtain a torque corresponding to each joint of the master manipulator according to the Cartesian force and a force Jacobian matrix of the master manipulator, and control the motor to output a resistance torque having a same magnitude as but an opposite direction to the torque, to make the Cartesian force of the mechanical arm perceived by an operator.

FIG. 1

FIG. 2

EP 3 892 226 A1

COLLISION
DETECTON SYSTEM          20

MECHANICAL ARM          21

FIG. 3

POSITION SENSOR          10

13          ALARM          11          PROCESSOR          14
                                                          DISCRETE
                                                          POINT
                                                          ARRANGING
                                                          UNIT

MEMORY          12

FIG. 4

20

FIG. 5

FIG. 6

4041

4042

404

404x

A

404n

FIG. 7

A-A

R$_{404x}$

FIG. 8

FIG. 9

FIG. 10

FIG. 11

| ARRANGING A PLURALITY OF DISCRETE POINTS ON EACH MECHANICAL ARM | —S10 |

↓

| ACQUIRING FIRST COORDINATE INFORMATION OF EACH DISCRETE POINT IN A GLOBAL COORDINATE SYSTEM | —S20 |

↓

| OBTAINING A FIRST RELATIVE DISTANCE BETWEEN TWO DISCRETE POINTS LOCATED ON DIFFERENT MECHANICAL ARMS ACCORDING TO THE FIRST COORDINATE INFORMATION | —S30 |

↓

| ACQUIRING A FIRST INTERACTION FORCE AT EACH DISCRETE POINT APPLIED BY EACH OF DISCRETE POINTS ON THE OTHER MECHANICAL ARM ACCORDING TO THE FIRST RELATIVE DISTANCE AND A CORRESPONDING RELATIONSHIP BETWEEN THE FIRST RELATIVE DISTANCE AND THE FIRST INTERACTION FORCE | —S40 |

↓

| CALCULATING A RESULTANT FORCE OF FIRST INTERACTION FORCES EACH OF WHICH CORRESPONDS TO EACH DISCRETE POINT AND EACH OF DISCRETE POINTS ON THE OTHER MECHANICAL ARM | —S50 |

↓

| OBTAINING A CARTESIAN FORCE OF EACH MECHANICAL ARM, AND MAKING THE CARTESIAN FORCE PERCEIVED BY AN OPERATOR | —S60 |

FIG. 12

<div align="center">

**INTERNATIONAL SEARCH REPORT**

</div>

| | International application No. |
|---|---|
| | **PCT/CN2019/119247** |

| | | |
|---|---|---|
| **A.** | **CLASSIFICATION OF SUBJECT MATTER** | |
| | A61B 34/30(2016.01)i;  A61B 34/00(2016.01)i | |
| | According to International Patent Classification (IPC) or to both national classification and IPC | |

| **B.** | **FIELDS SEARCHED** |
|---|---|
| Minimum documentation searched (classification system followed by classification symbols) | |
| A61B | |
| Documentation searched other than minimum documentation to the extent that such documents are included in the fields searched | |
| Electronic data base consulted during the international search (name of data base and, where practicable, search terms used) | |
| CNABS, EPODOC, WPI, CNKI: 微创, 机器人, 机械臂, 碰撞, 撞击, 相撞, 离散, 点, 体积, 轮廓, 中心, 坐标, 距离, 力, 关系, 函数, robot, surgica+, arm, collid+, collis+, block, contour, center, coordinate, distance, force, function | |

| **C.** | **DOCUMENTS CONSIDERED TO BE RELEVANT** | |
|---|---|---|
| Category* | Citation of document, with indication, where appropriate, of the relevant passages | Relevant to claim No. |
| PX | CN 109620410 A (MICROPORT (SHANGHAI) MEDICAL ROBOT CO., LTD.) 16 April 2019 (2019-04-16) description, paragraphs [0089]-[0141], claims 1-21, and figures 1-12 | 1-22 |
| A | CN 102448680 A (INTUITIVE SURGICAL, INC.) 09 May 2012 (2012-05-09) description, paragraphs [0032]-[0096], and figures 1-14 | 1-22 |
| A | CN 107595392 A (INTUITIVE SURGICAL, INC.) 19 January 2018 (2018-01-19) entire document | 1-22 |
| A | CN 106725861 A (SHANDONG UNIVERSITY) 31 May 2017 (2017-05-31) entire document | 1-22 |
| A | CN 107645924 A (MOBIUS IMAGING LLC) 30 January 2018 (2018-01-30) entire document | 1-22 |
| A | US 2017189131 A1 (ETHICON ENDO-SURGERY, LLC) 06 July 2017 (2017-07-06) entire document | 1-22 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 January 2020** | **31 January 2020** |

| Name and mailing address of the ISA/CN | Authorized officer |
|---|---|
| **China National Intellectual Property Administration (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2019/119247**

| Patent document cited in search report | | | Publication date (day/month/year) | Patent family member(s) | | | Publication date (day/month/year) |
|---|---|---|---|---|---|---|---|
| CN | 109620410 | A | 16 April 2019 | None | | | |
| CN | 102448680 | A | 09 May 2012 | JP | 2016073686 | A | 12 May 2016 |
| | | | | EP | 2414137 | B1 | 21 June 2017 |
| | | | | US | 2017282372 | A1 | 05 October 2017 |
| | | | | JP | 5840121 | B2 | 06 January 2016 |
| | | | | US | 2018126559 | A9 | 10 May 2018 |
| | | | | JP | 2014097431 | A | 29 May 2014 |
| | | | | JP | 6576002 | B2 | 18 September 2019 |
| | | | | WO | 2010117685 | A2 | 14 October 2010 |
| | | | | KR | 101705921 | B1 | 10 February 2017 |
| | | | | JP | 2016052521 | A | 14 April 2016 |
| | | | | JP | 2016064155 | A | 28 April 2016 |
| | | | | CN | 102448680 | B | 27 April 2016 |
| | | | | KR | 20120004479 | A | 12 January 2012 |
| | | | | JP | 2016101506 | A | 02 June 2016 |
| | | | | JP | 2019202158 | A | 28 November 2019 |
| | | | | EP | 2414137 | A2 | 08 February 2012 |
| | | | | JP | 6058111 | B2 | 11 January 2017 |
| | | | | EP | 3385039 | A1 | 10 October 2018 |
| | | | | EP | 3246135 | A1 | 22 November 2017 |
| | | | | JP | 2012521855 | A | 20 September 2012 |
| | | | | US | 2019047154 | A1 | 14 February 2019 |
| | | | | US | 2009192524 | A1 | 30 July 2009 |
| | | | | JP | 2017176848 | A | 05 October 2017 |
| | | | | US | 10137575 | B2 | 27 November 2018 |
| | | | | US | 9789608 | B2 | 17 October 2017 |
| | | | | EP | 3385039 | B1 | 06 November 2019 |
| | | | | EP | 2326277 | B1 | 21 March 2018 |
| | | | | JP | 2011525845 | A | 29 September 2011 |
| | | | | JP | 5583123 | B2 | 03 September 2014 |
| | | | | JP | 2015051287 | A | 19 March 2015 |
| | | | | EP | 2326277 | A1 | 01 June 2011 |
| | | | | EP | 3351203 | A1 | 25 July 2018 |
| | | | | JP | 2014012212 | A | 23 January 2014 |
| CN | 107595392 | A | 19 January 2018 | JP | 2015521084 | A | 27 July 2015 |
| | | | | US | 9492235 | B2 | 15 November 2016 |
| | | | | US | 2013325029 | A1 | 05 December 2013 |
| | | | | CN | 104334110 | B | 03 October 2017 |
| | | | | KR | 20150023359 | A | 05 March 2015 |
| | | | | CN | 104334110 | A | 04 February 2015 |
| | | | | JP | 2018158155 | A | 11 October 2018 |
| | | | | WO | 2013181503 | A1 | 05 December 2013 |
| | | | | EP | 2854688 | A1 | 08 April 2015 |
| | | | | JP | 6368710 | B2 | 01 August 2018 |
| CN | 106725861 | A | 31 May 2017 | CN | 106725861 | B | 10 December 2019 |
| CN | 107645924 | A | 30 January 2018 | WO | 2016168671 | A1 | 20 October 2016 |
| | | | | US | 2016302871 | A1 | 20 October 2016 |
| | | | | EP | 3282997 | A1 | 21 February 2018 |
| US | 2017189131 | A1 | 06 July 2017 | US | 10154886 | B2 | 18 December 2018 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201811472969